# EUROPEAN PATENT SPECIFICATION

(11) **EP 4 299 193 B1**
(45) Date of publication and mention of the grant of the patent: **08.07.2026**
(21) Application number: 22868416.3
(22) Date of filing: 18.11.2022
(51) Int. Cl.: B05C 3/12, B05C 3/15, B05C 11/02, B05C 13/02, G01N 33/531

(54) **MEMBRANE TREATMENT APPARATUS**
MEMBRANBEHANDLUNGSVORRICHTUNG
APPAREIL DE TRAITEMENT À MEMBRANES

(30) Priority: 18.05.2022 CN 202210536168
(43) Date of publication of application: 03.01.2024
(73) Proprietor: Leadway (HK) Limited, Sheung Wan, Hong Kong 999077 (CN)
(72) Inventor: CUI, Chao, Zhejiang 310030 (CN)
(74) Representative: Schiweck Weinzierl Koch Patentanwälte Partnerschaft mbB
(86) International application number: PCT/CN2022/132935
(87) International publication number: WO 2023/221436

(56) References cited:
- CN-A- 109 688 993
- CN-A- 109 759 275
- CN-A- 114 632 666
- CN-B- 109 759 275
- CN-U- 206 985 303
- CN-U- 206 985 303
- CN-U- 213 349 500
- JP-A- 2004 314 332
- JP-A- 2007 294 598

## Description

### Field of the Invention

The present invention relates to a production apparatus, in particular to an apparatus for processing a film by means of pressure, said film being available for manufacturing of test paper.

### Background of the Invention

Disposable detection kits for detection of diseases or other physical conditions using body fluids such as urine, blood or other tissue fluids of a human body have been commonly used all over the world, and their applications can be in laboratories operated by professionals, or at home, schools, shopping malls, road checkpoints, customs, etc. operated by non-professionals themselves. Such a detection kit generally includes an upper cover, a bottom plate and a test strip, wherein the upper cover and the bottom plate are combined in such a manner as buckling, welding, glue adhering or the like, and the test strip is arranged on the bottom plate and located between the upper cover and the bottom plate. The upper cover includes a sample addition hole and an observation hole. A liquid sample for detection is applied to a test strip via the sample addition hole, and after the end of the detection, a detection result on the test strip is observed via the observation hole, and determined accordingly.

The test strip generally includes a rigid bottom card, and a sample addition pad, a marker pad, a detection pad and a water absorbing pad which are affixed to the rigid bottom card, successively spliced end to end, and all made of a water-absorbing film material. The label pad is labeled with labels for color development in advance, the detection pad is provided with a detection line and a quality control line, and the water absorbing pad has better water absorbing performances than the other three pads, thereby attracting a liquid sample dropwise added to the sample addition pad to diffuse from the sample addition pad to the label pad, the detection pad and the water absorbing pad sequentially. In the above diffusion process, the liquid sample dissolves the labels for color development on the label pad at first, and brings these labels for color development to diffuse downstream. When the liquid sample flows through the detection line, if there are sufficient components to be detected in the liquid sample, then these components to be detected and the labels for color development are captured together by substances on the detection line and accumulated in the detection line area. When a sufficient amount of these components to be detected and labels for color development have been accumulated in the detection line area, the detection line area displays a color. A positive test result is obtained. The liquid solution continues to flow downstream to reach the quality control line area and displays a color in the quality control line area, indicating the detection result is valid. Regardless of whether the detection line area displays a color, it indicates the detection result is invalid as long as the quality control line area does not display a color. If the quality control line area displays a color, but the detection line area does not display a color, it indicates the detection result is negative; and if the quality control line area displays a color and the test line area also displays a color, it indicates the detection result is positive. Taking the double antibody sandwich method as an example, a darker color of the detection line area indicates a stronger positive signal and a higher content or concentration of the substance to be detected; and on the contrary, a lighter color of the detection line area indicates a weaker positive signal and a lower content or concentration of the substance to be detected, and it is necessary to take another sample for re-detection after a period of time.

The film for manufacturing the sample addition pad, the label pad, the detection pad and the water absorbing pad is generally a porous water-absorbing material, and needs processing to obtain its required performances. The soaking method is a common film processing method. The soaking method in the prior art generally includes unfolding the film, followed by soaking it in a container containing a chemical reagent solution on a soaking apparatus for appropriate time, and then airing or drying the film after picking it up. However, the existing soaking apparatus cannot be well adapted to films of different materials, thicknesses or softnesses. In other words, when a new film is replaced for use, performance parameters of the new film are slightly different from those of the previous batch of films, which results in that the existing soaking apparatus has to undergo a long period of debugging before it can be well adapted to the new film. In addition, after the apparatus has been running for a period of time, some parts usually have deviations in position, so it is necessary to find the initial position again. However, the existing apparatus does not have the function of automatically finding the initial position, and it requires to manually debug the apparatus to find the initial position, which is laborious and inefficient. Moreover, in the debugging process, the existing apparatus cannot carry out production and is in a shut-down state, thus reducing the production efficiency.

An objective of the present invention is to provide a film processing apparatus, which can automatically adjust the state thereof according to the input parameter, so that the apparatus can be quickly adapted to different films, which greatly reduces the time required by debugging the existing apparatus for different films, and in turn greatly shortens the downtime of the apparatus, thus greatly improving the production efficiency of the apparatus.

The present invention is proposed in such background.

Conventional apparatuses and methods for processing a film are disclosed in JP 2007 294598 A.

### Summary of the Invention

In order to solve the above technical problems, an apparatus for processing a film is provided according to claim 1. The invention is defined by the appended set of claims. The description that follows is subjected to this limitation. Any disclosure lying outside the scope of said claims is only intended for illustrative as well as comparative purposes. Further improvement of the present invention lies in that the optocoupler switch comprises a U-shaped groove; the scraping mechanism comprises an opaque paddle; and when the paddle enters into the U-shaped groove, the optocoupler switch is triggered to emit a signal, the stepping motor stops running, and the first scraping shaft and the second scraping shaft are in initial positions.

Further improvement of the present invention lies in that the scraping mechanism further comprises a synchronizing gear, the stepping motor drives the scraping mechanism to rotate together via the synchronizing gear, so as to adjust the included angle between the connecting line of the axes of the first scraping shaft and the second scraping shaft and the vertical line.

Further improvement of the present invention lies in that the paddle is fixed to the side face of the synchronizing gear.

Further improvement of the present invention lies in that the feeding shaft comprises two roll wheels, at least one of which is connected with an elastic element and swingable relative to the stand.

Further improvement of the present invention lies in that the feeding shaft comprises two roll wheels, and a counterweight element movable up and down is disposed between the two roll wheels.

Further improvement of the present invention lies in that the feeding shaft further comprises a wing extending downwards, a guide slot is disposed on the wing, and the counterweight element is located in the guide slot and movable up and down in the guide slot.

According to the invention, the appropriate distance provides a space for passage of the film between the first scraping shaft and the second scarping shaft, so that the upper and lower surfaces of the film abut against the outer surfaces of the first scraping shaft and the second scarping shaft, respectively, thus the first scraping shaft and the second scraping shaft generate a squeezing or scraping force corresponding to the input parameter on the film.

Further improvement of the present invention lies in that the scraping mechanism comprises a first baffle and a second baffle, to which two ends of the first scraping shaft and the second scraping shaft are connected respectively; the first baffle and the second baffle are connected to the stand, respectively; the first baffle is connected to the synchronizing gear which is connected to the stepping motor; and under the driving of the stepping motor, the synchronizing gear drives the first baffle and the second baffle together with the first scraping shaft and the second scraping shaft to rotate synchronously relative to the stand, so as to adjust the included angle between the connecting line of the axes of the first scraping shaft and the second scraping shaft and the vertical line.

Further improvement of the present invention lies in that the film is used for manufacturing of test paper.

### Beneficial effects

The present invention has the beneficial effects that the film processing apparatus in the present invention has the function of automatically finding the initial position and is capable of quickly adjusting the state of the apparatus according to the input parameter, so that the apparatus can quickly apply to the new film replaced for use, which greatly shortens the debugging time of the apparatus and greatly reduces the downtime of the apparatus, and thus greatly improves the production efficiency of the apparatus. Moreover, the apparatus in the present invention allows passage of the film between the first scraping shaft and the second scraping shaft, and the first scraping shaft and the second scraping shaft generate a squeezing or scraping force on the film abutting against the shafts to remove excess chemical reagent solution on the film, so that the chemical reagent solution is evenly distributed on the film. The present invention has the advantages of simple operation, high production efficiency, low cost, wide application range, etc. The prepared film can be used for manufacturing test paper, improving the performance of the test paper.

### Brief Description of the Drawings

Fig. 1 is a stereoscopic schematic view of an apparatus for processing a film used for manufacturing of test paper (hereinafter referred to as "film processing apparatus");
Fig. 2 is a stereoscopic schematic view of Fig. 1 from another perspective;
Fig. 3 is a planar schematic view of Fig. 2;
Fig. 4 is a planar schematic view of the back side of Fig. 3;
Fig. 5 is a planar schematic view of a modified embodiment of Fig. 4;
Fig. 6 is an enlarged schematic view of a part within circle A in Fig. 1;
Fig. 7 is a stereoscopic exploded schematic view of test paper manufactured using a film processed by the film processing apparatus in the present invention;
Fig. 8 is a stereoscopic combined view of Fig. 7;
Fig. 9 is a stereoscopic exploded schematic view of performing sealed package on the test paper shown in Fig. 8 in a first manner;
Fig. 10 is a planar schematic view of a detection device formed after performing sealed package on the test paper shown in Fig. 8;
Fig. 11 is a sectional view along line A-A of Fig. 10;
Fig. 12 is a planar schematic view of a multi-lined detection device consisting of ten detection devices shown in Fig. 10 arranged side by side, with two adjacent detection devices being connected by a easy-tear line;
Fig. 13 is a sectional view along line B-B of Fig. 12;
Fig. 14 is a stereoscopic exploded schematic view of assembling the test paper shown in Fig. 8 in a second manner;
Fig. 15 is a stereoscopic combined view of Fig. 14;
Fig. 16 is a state of a scraping mechanism in the initial position;
Fig. 17 is a state of the scraping mechanism after automatic adjustment after the first batch of films are mounted to the film processing apparatus in the present invention; and
Fig. 18 is a state of the scraping mechanism after automatic adjustment after the second batch of films are mounted to the film processing apparatus in the present invention.

### Detailed Description of the Embodiments

The technical solutions of the present invention will be further specified below through embodiments in conjunction with the drawings.

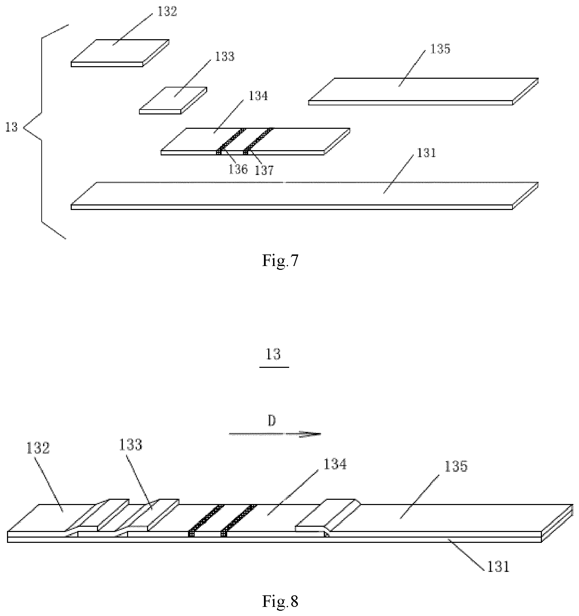

Test paper 13, as shown in Figs. 7 and 8, is lateral flow test paper. The test paper 13 includes a bottom plate 131, to which a sample pad 132, a label binding pad 133, a test pad 134 and a water absorbing pad 135 are successively lapped each other and adhered from upstream to downstream (i.e., the flow direction of the added liquid sample to be detected or the mixture of the liquid sample and a buffer on the test paper, i.e., the direction indicated by arrow D in Fig. 8). The label binding pad 133 includes labels that can be bonded to analyte, for example, latex labeled with antigens or antibodies, colloidal gold, fluorescent microspheres, etc. The test pad 134 is generally provided with a detection line 136 and a quality control line 137.

Different types of samples are selected according to different detection items. For example, the sample for detection of SARS-CoV-2 is a sample collected by a throat swab or a nose swab, the sample for examination of pregnancy is urine, the sample for detection of blood fat is blood, etc. Test paper with different principles and structures can also be selected according to different detection items.

The sample pad 132, the label binding pad 133, the test pad 134 and the water absorbing pad 135 are generally made of a water-absorbing film material. For example, the material of the sample pad 132 is selected from glass cellulose, polyester film, polyester fiber film, non-woven fabric, and filter paper; the material of the label binding pad 133 is selected from glass cellulose, polyester film, polyester fiber film, and non-woven fabric; the material of the test pad 134 is selected from NC film, and nitrocellulose film; and the material of the water absorbing pad 135 is selected from cellulose filter paper, absorbent paper, and filter paper. For ease of illustration, these film materials are collectively referred to as films in the present invention. Moreover, the material of the bottom plate 131 is selected from a hydrophobic material such as polyester (PET) adhesive plate, polyvinyl chloride (PVC) adhesive plate, polypropylene (PP) adhesive plate, and polycarbonate (PC) adhesive plate. The material of the the bottom plate 131 is different from the above film materials.

In the actual production process, these films usually need to be processed with some suitable chemical reagents to obtain better performances. Taking the water absorbing pad 135 as an example, the water absorbing pad 135 usually has to have better water absorbing performances than the other three pads in order to urge the liquid sample added to the sample pad 132 to diffuse rapidly from the sample pad 132 to the label binding pad 133, the test pad 134 and the water absorbing pad 135 in sequence, so as to achieve the purpose of quickly completing the detection. To this end, the water absorbing pad 135, in addition to being made of a material with superior water absorbing performances, usually requires to undergo some processing to obtain better water absorbing performances and other necessary performances. The processing method includes first soaking the film in a prepared solution, followed by drying the film, and then cutting the film to have proper size. Next these films are spliced and affixed to the bottom plate 131 in a lap end to end manner according to the desired order, and then cut into test paper 13 of proper size (as shown in Fig. 8).

The present invention provides an apparatus for processing a film for manufacturing of test paper. The films processed using the present invention can be processed into the sample pad 132, the label binding pad 133, the test pad 134, and the water absorbing pad 135, respectively. These films processed using the present invention may also require other processing, and those other processing methods or apparatuses do not belong to the contents of the present invention, and thus are not described in detail in the present invention. In addition, processing different films by using the present invention may also require the use of solutions prepared with different chemical reagents. The chemical reagent formulations of these solutions are the prior art in the field and do not belong to the contents of the present invention, and thus are not described in detail in the present invention; and they are replaced only with reagents, liquid reagents, chemical reagents or chemical reagent solutions for the sake of concision.

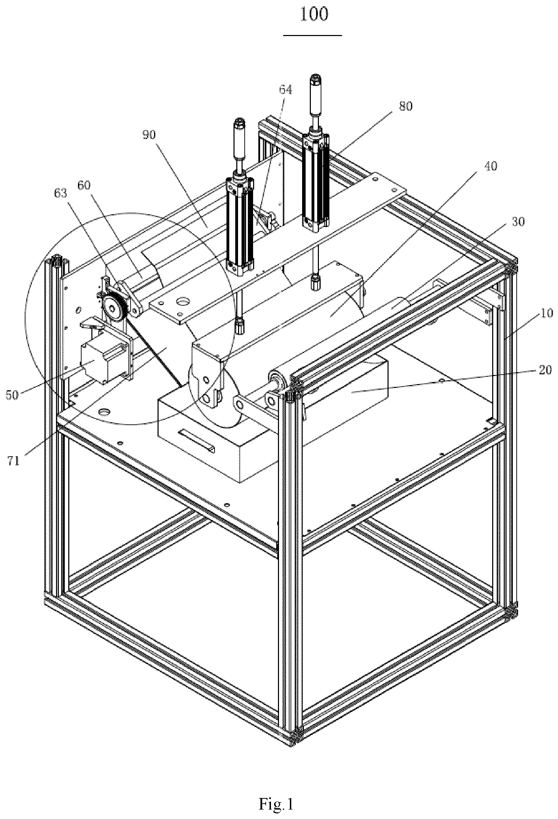

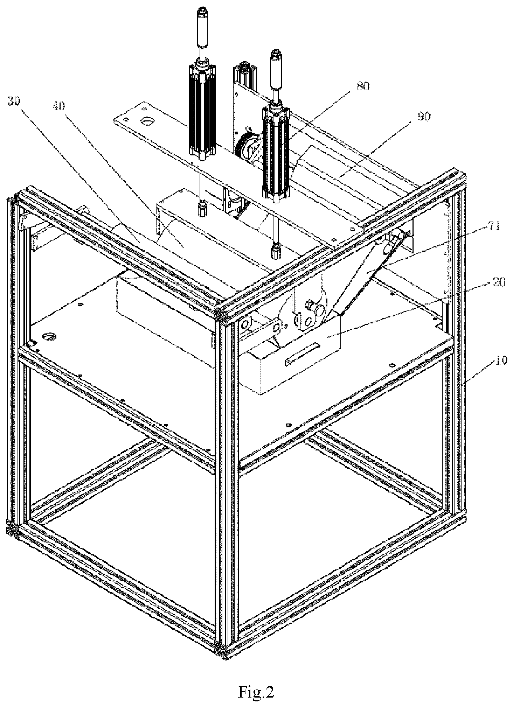

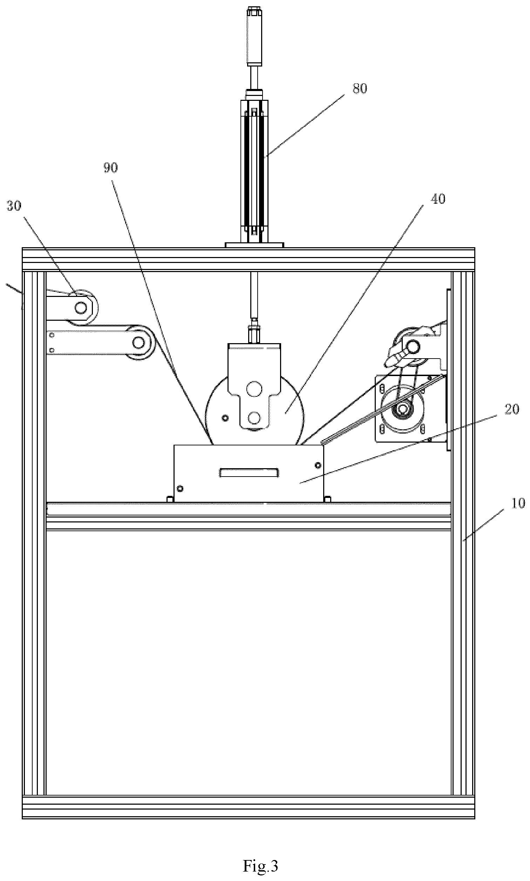

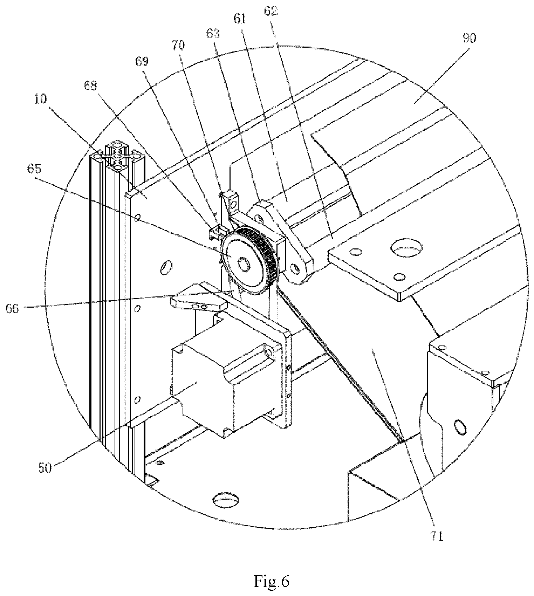

Referring to Fig. 1 to Fig. 6, an apparatus 100 for processing a film for manufacturing of test paper of the present invention (hereinafter referred to as "film processing apparatus" or "apparatus of the present invention") includes a stand 10, a soaking tank 20, as well as a feeding shaft 30, a roll shaft 40, a stepping motor 50, and a scraping mechanism 60 mounted to the stand 10, respectively. Please refer to Fig. 1, the roll shaft 40 is of a cylindrical structure, which can rotate around its shaft. The roll shaft 40 can enter or go out of the soaking tank 20 under the driving of an air cylinder piston 80 (which may be a hydraulic cylinder piston or a mechanical arm or the like, wherein two air cylinder pistons are shown in the figure). The soaking tank 20 contains an appropriate amount of chemical reagent solution. When the roll shaft 40 enters into the soaking tank 20, the lower part of the cylindrical outer surface of the roll shaft 40 pushes the film 90 to be below the liquid level of the chemical reagent solution in the soaking tank 20, so that the film 90 is fully soaked in the chemical reagent solution, thus absorbing a part of the chemical reagent solution onto the film. The feeding shaft 30 is used to convey the film 90 into the film processing apparatus of the present invention and to keep the film 90 in a sufficiently stretched state, so as to prevent the film from being turned over, folded or snapped, which affects the automated production. The scraping mechanism 60 is used to remove excess chemical reagent solution from the film 90 which gets out of the soaking tank 20 and has absorbed enough chemical reagent solution (a recovery plate 71 is mounted under the film 90 to recover the chemical reagent solution falling off the film 90) for facilitating subsequent drying operations, and to improve the uniformity of chemical reagent solution absorption in various areas on the film 90, thereby improving the homogeneity of the performances of the whole film, which is very important to improve the yield of the test paper. As shown in Figs. 1 and 6, the scraping mechanism 60 includes a first scraping shaft 61 and a second scraping shaft 62 arranged side by side and spaced from each other at an appropriate distance, and a synchronizing gear 65. Both the first scraping shaft 61 and the second scraping shaft 62 are of cylindrical structures (including a hollow cylindrical barrel structure and a solid cylindrical bar structure), and their two ends are connected to the first baffle 63 and the second baffle 64, respectively. The first baffle 63 and the second baffle 64 are connected to the stand 10 respectively and are rotatable relative to the stand. The first baffle 63 is connected to the synchronizing gear 65 and rotates synchronously with the synchronizing gear 65. The synchronizing gear 65 is connected to the stepping motor 50 by a gear belt 66, and under the driving of the stepping motor 50, the synchronizing gear 65 drives the first baffle 63 and the second baffle 64 together with the first scraping shaft 61 and the second scraping shaft 62 to jointly rotate around the axis of the synchronizing gear 65. When the film 90 is processed by the apparatus of the present invention, the film 90 is passed between the first scraping shaft 61 and the second scraping shaft 62, so that the upper and lower surfaces of the film 90 abut against the outer surfaces of the first scraping shaft 61 and the second scraping shaft 62, respectively, and the segment of the film 90 between the first scraping shaft 61 and the second scraping shaft 62 has an appropriate included angle with respect to the two segments adjacent to this segment (the appropriate included angle may be determined experimentally according to the material, thickness, flexibility, strength and other performances of the film) to ensure that the first scraping shaft 61 and the second scraping shaft 62 generate an appropriate squeezing or scraping force on the film 90.

With the appropriate squeezing or scraping force, excess chemical reagent solution on the soaked film 90 can be removed, and the chemical reagent solution absorbed by the film 90 is distributed as evenly as possible on the film 90.

Please refer to Fig. 6, an optocoupler switch 68 is fixed to the stand 10 at an appropriate position near the synchronizing gear 65. The optocoupler switch 68 includes a U-shaped groove 69. An opaque paddle 70 is fixed to the side face of the synchronizing gear 65. The synchronizing gear 65 is driven by the stepping motor 50 to rotate, which in turn drives the paddle 70 to rotate synchronously. When the paddle 70 enters the U-shaped groove 69, the paddle 70 cuts off the optical path of the optocoupler switch 68, so that the optocoupler switch 68 is triggered to emit a signal, the stepping motor 50 stops running, and this position is recorded as the initial position. In the present invention, the above process is referred to as the process that the optocoupler switch 68 automatically finds the initial position. Then, this initial position is taken as the starting point, and then the stepping motor 50 rotates an appropriate angle according to an input parameter (the input parameter can be determined experimentally in laboratories according to the material, thickness, flexibility, strength and other performances of the films by randomly selecting a small portion from the large batch of films used for production (for example, a roll of film selected from multiple rolls of films in the same batch, or a segment taken from a roll of film), thus not affecting the normal running of the film processing apparatus in a production workshop), which in turn drives the scraping mechanism 60 to swing an angle corresponding to the input parameter, that is, the connecting line of the axes of the first scraping shaft 61 and the second scraping shaft 62 deflects, by taking the axis of the synchronizing gear 65 as a pivot, an angle corresponding to the input parameter, so that the outer surfaces of the first scraping shaft 61 and the second scraping shaft 62 generate an appropriate squeezing or scraping force on the film 90. In the present invention, the above process is referred to as automatically adjusting the position and angle of the scraping mechanism 60, or further referred to as adjusting the positions and angles of the first scraping shaft 61 and the second scraping shaft 62. The optocoupler switch 68 of the present invention is also referred to as a photoelectric sensor, which can select a Model H2010 or GA105A direct-emitting photoelectric sensor with trademark TAO TiME CLUB^{®}; and this photoelectric sensor is available on Jingdong Mall (WWW.JD.COM).

It should be noted that the stepping motor 50 of the present invention runs only when it automatically finds the initial position and adjusts the positions and angles of the synchronizing gear 65 and the scraping mechanism 60 according to the input parameter, and stays in a fixed position at all other times without running. When a new film is replaced for use, the stepping motor 50 is restarted to automatically find the initial position again and automatically readjust the positions and angles of the synchronizing gear 65 and the scraping mechanism 60 according to the input parameter, and then keeps unchanged in this fixed position, and the film processing apparatus 100 of the present invention starts to run normally.

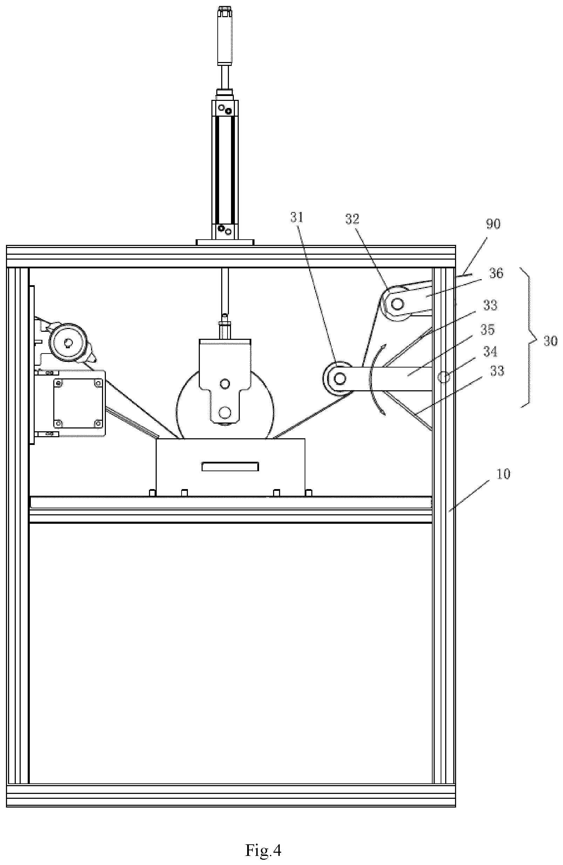

In the embodiment as shown in Fig. 4, the feeding shaft 30 includes a first roll wheel 31 and a second roll wheel 32, which are connected to the stand 10 by a first cantilever 35 and a second cantilever 36, respectively. The first cantilever 35 is connected to an elastic element 33, and is rotatable about a support shaft 34 to which the stand 10 is movably connected. Wherein, two stretched elastic elements 33 are shown in Fig. 4, and pull the first cantilever 35 from the upper and lower sides of the first cantilever 35, respectively, so that the first cantilever 35 can swing up and down with the support shaft 34 as the pivot (as shown by the direction of the arrow in Fig. 4). The second cantilever 36 is fixedly connected to the stand 10. The elastic element 33 is used to control the swing angle of the first cantilever 35, and thus the position relationship between the first roll wheel 31 and the second roll wheel 32. When film processing is performed by the apparatus 100 of the present invention, the film 90 is passed between the first roll wheel 31 and the second roll wheel 32, so that the upper and lower surfaces of the film 90 abut against the outer surfaces of the first roll wheel 31 and the second roll wheel 32, respectively, and the segment of the film 90 between the first roll wheel 31 and the second roll wheel 32 has an appropriate included angle with respect to the two segments adjacent to this segment (the appropriate included angle may be determined experimentally according to the material, thickness, flexibility, strength and other performances of the film) to ensure that the first roll wheel 31 and the second roll wheel 32 generate an appropriate squeezing or scraping force on the film 90, thereby ensuring that the film 90 is always in a moderate stretched state; and then the film 90 smoothly enters into the soaking tank 20 by squeezing of the roll shaft 40 and thus is sufficiently soaked by the chemical reagent solution in the soaking tank 20. In another embodiment, it is also possible to design the first cantilever 35 to be fixedly connected to the stand 10 and design the second cantilever 36 to be movably connected to the stand 10, and to control the swing of the second cantilever 36 by the elastic element 33. In yet another embodiment, it is also possible to design both the first cantilever 35 and the second cantilever 36 to be movably connected to the stand 10, and to control the swing of both the first cantilever 35 and the second cantilever 36 by the elastic element 33 (not shown in the figure). The elastic element 33 of the present invention may be selected from an extension spring, a rubber band, or other suitable elastic element known to those skilled in the art.

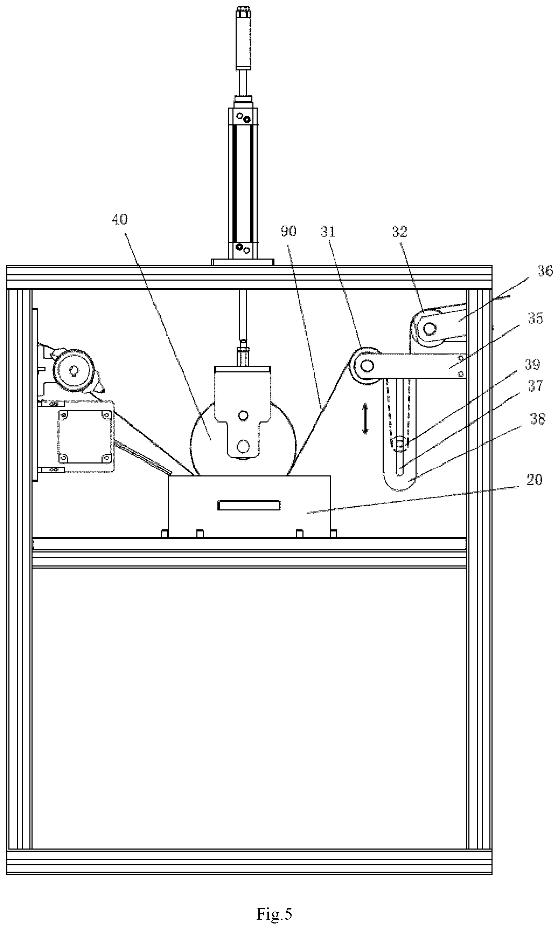

Fig. 5 shows another embodiment that differs slightly from Fig. 4. The embodiment shown in Fig. 5 differs from the embodiment shown in Fig. 4 in that both the first cantilever 35 and the second cantilever 36 are connected to the stand 10, a wing 38 with a guide slot 37 is fixedly connected below the first cantilever 35, and the wing 38 is located between the first roll wheel 31 and the second roll wheel 32. A counterweight element 39 is received in the guide slot 37, and is movable up and down in the guide slot 37 (indicated by the direction of the arrow in Fig. 5). When film processing is performed by the apparatus 100 of the present invention, the film 90 is passed between the first roll wheel 31 and the second roll wheel 32 so that the upper and lower surfaces of the film 90 abut against the outer surfaces of the first roll wheel 31 and the second roll wheel 32, respectively, and the film 90 passes through the counterweight element 39 to be pulled downward by means of the action of gravity of the counterweight element 39 itself to ensure that the film 90 is always in a moderate stretched state; and then the film 90 smoothly enters into the soaking tank 20 by squeezing of the roll shaft 40 and thus is sufficiently soaked by the chemical reagent solution in the soaking tank 20. The weight of the counterweight element 39 can be determined experimentally, and it will be better to ensure that the film 90 is always in a moderate stretched state without damaging the film 90.

In the preferred embodiment of the present invention, a power mechanism (not shown in the figure) is also provided in front of the scraping mechanism 60 to pull the film so that the film is pulled to proceed passing by the feeding shaft 30, the roll shaft 40 and the scraping mechanism 60 in sequence on the apparatus.

The method of processing the film 90 using the film processing apparatus 100 of the present invention comprises the following steps:
A, manipulating the air cylinder piston 80 so that the roll shaft 40 is outside of the soaking tank 20;
B, passing the film 90 sequentially through the feeding shaft 30, the roll shaft 40 and the scraping mechanism 60, so that the film 90 sequentially abuts against the outer cylindrical surfaces of the first roll wheel 31, the second wheel 32, the roll shaft 40, the first scraping shaft 61 and the second scraping shaft 62 and keeps being appropriately stretched;
C, injecting an appropriate amount of chemical reagent solution into the soaking tank 20;
D, manipulating the air cylinder piston 80, so that the roll shaft 40 enters into the soaking tank 20 and the film 90 under the roll shaft 40 is soaked in the chemical reagent solution in the soaking tank 20;
E, starting the stepping motor 50, driving, by the stepping motor 50, the synchronizing gear 65 and the paddle 70 fixed to the side face of the synchronizing gear 65 to rotate synchronously, and when the paddle 70 enters the U-shaped groove 69 of the optocoupler switch 68, cutting off, by the paddle 70, the optical path of the optocoupler switch 68, stopping running of the stepping motor 50, and recording this position as the initial position;
F, inputting a parameter, restarting the stepping motor 50, driving, by the stepping motor 50, the scraping mechanism 60 to swing a rotation angle corresponding to the input parameter according to the input parameter, followed by stopping running of the stepping motor, and keeping the scraping mechanism 60 unchanged in this working position; and
G, continuously conveying, by the feeding shaft 30, the film 90 to the roll shaft 40 and the scraping mechanism 60.

Figs. 7 and 8 show test paper 13 (with its structure as previously stated) manufactured using the processed film 90 after the film 90 is processed using the film processing apparatus 100 and the film processing method of the present invention.

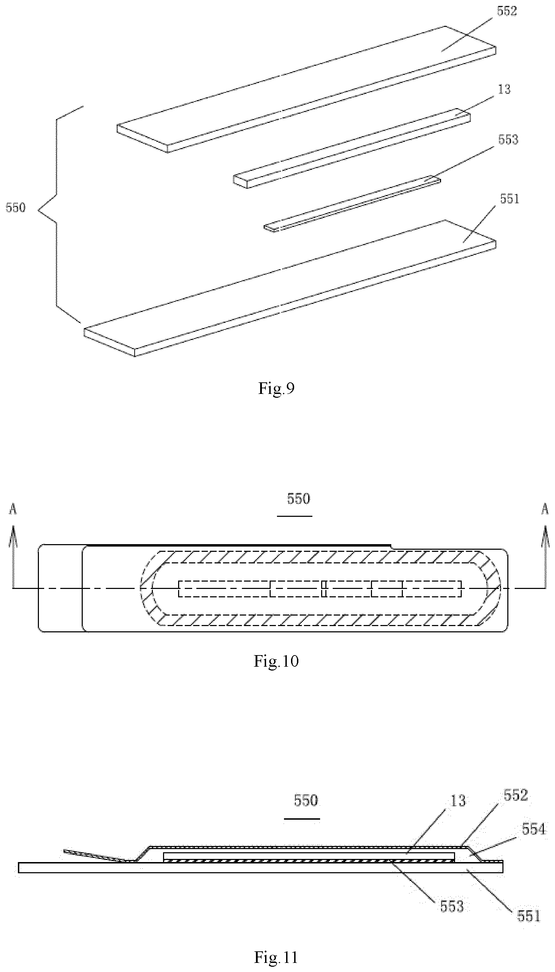

Figs. 9 to 11 show a first type of detection device 550 manufactured using the test paper 13 described above. The detection device 550 includes a rigid substrate 551, the test paper 13, and a sealing film 552. Wherein, the test paper 13 is bonded and fixed to the rigid substrate 551 by a bonder 553, the edge of the sealing film 552 is bonded to the rigid substrate 551 using a bonder or a hot melt process, and the test paper 13 is sealed and mounted in the cavity 554 between the rigid substrate 551 and the sealing film 552.

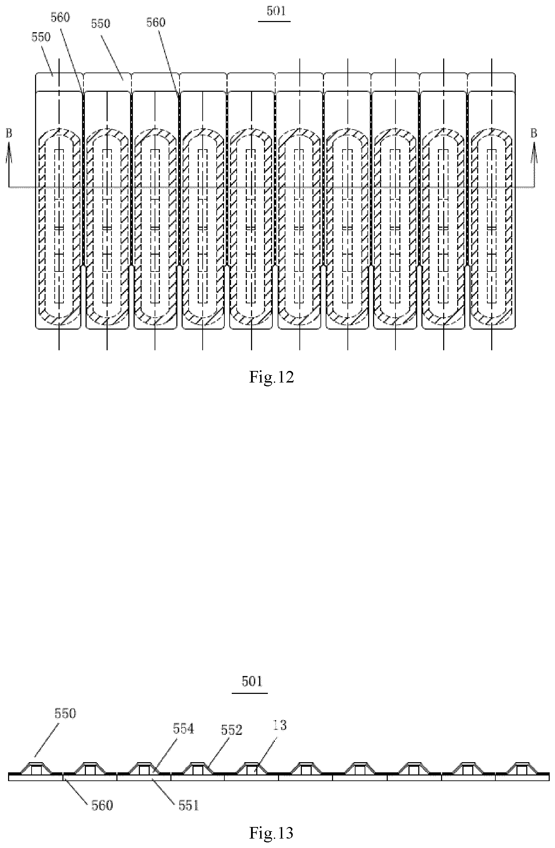

Figs. 12 and 13 show a multi-lined detection device 501 with ten detection devices 550 shown in Fig. 10 connected side by side in a row. Wherein, the connecting portion between two adjacent detection devices 550 is an easy-tear line 560 so as to conveniently tear off the detection devices 550 connected in a row for the detection operation. In the subsequent detection operation, the sealing film 552 is first torn off from the rigid substrate 551 to completely expose the test paper 13, and then the liquid sample to be detected is added according to the existing method, and the detection result is observed after waiting for a certain period of time. The first type of detection device 550 and the multi-lined detection device 501 have the advantages that it is easy to realize automated production, the product yield is high, it is convenient to use and the production cost is low.

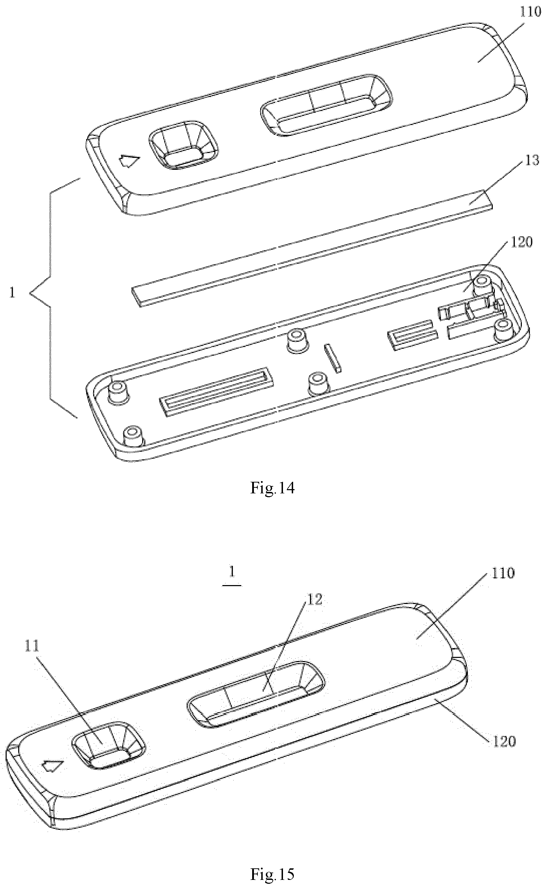

Figs. 14 to 15 show a second type of detection device 1 manufactured using the test paper 13 described above. The detection device 1 includes an upper cover 110, a bottom plate 120 and test paper 13, wherein the test paper 13 is fixed in the bottom plate 120 and the upper cover 110 covers the bottom plate 120. The upper cover 110 is provided with a sample addition hole 11 and a detection result observation window 12 corresponding to the sample pad 132 and test pad 134 of the test paper 13, respectively.

The liquid sample is added from the sample addition hole 11. After waiting for a certain period of time (taking the detection of novel coronavirus as an example, the best waiting time is fifteen to thirty minutes after addition of the liquid sample, and the detection result observed within this period of time has the highest accuracy), color change in the detection result observation window 12 is observed, and the detection result is determined according to the existing methods.

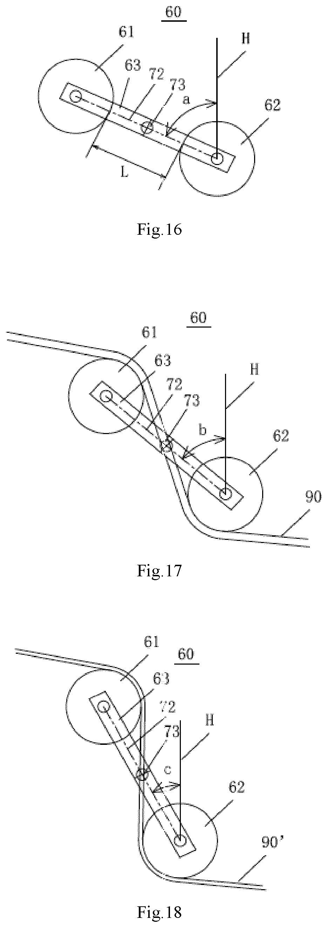

Figs. 16 to 18 show the automatic adjustment process of the scraping mechanism 60. In Fig. 16, with the assistance of the optocoupler switch 68, the stepping motor 50 drives the scraping mechanism 60 to be in the initial position, and at this point, the included angle between the connecting line 72 of the axes of the first scraping shaft 61 and the second scraping shaft 62 and a vertical line "H" is "a". Fig. 17 shows that after the first batch of films 90 are mounted to the film processing apparatus 100 of the present invention, the film processing apparatus 100, based on a parameter that is input by an operator and corresponds to the first batch of films 90 (as described previously, the parameter can be obtained by limited times of measurements or experiments on the first batch of films 90, the same below), causes the stepping motor 50 to drive the scraping mechanism 60, with the connecting shaft 73 of the scraping mechanism 60 and the stand 10 as a shaft, to rotate an angle corresponding to the input parameter (the corresponding relationship between this angle and the input parameter can be obtained through limited times of measurements or experiments on the first batch of films 90, the same below), so that the included angle between the connecting line 72 of the axes of the first scraping shaft 61 and the second scraping shaft 62 and the vertical line "H" becomes "b" to realize the automatic adjustment function, and thus the first scraping shaft 61 and the second scraping shaft 62 generate a squeezing or scraping force corresponding to the input parameter on the film 90 (the corresponding relationship between the magnitude of the squeezing or scraping force and the input parameter can be obtained through limited times of measurements or experiments, the same below). Fig. 18 shows that after the second batch of films 90' are mounted to the film processing apparatus 100 of the present invention, the film processing apparatus 100, based on a parameter that is input by an operator and corresponds to the second batch of films 90', causes the stepping motor 50 to drive the scraping mechanism 60, with the connecting shaft 73 of the scraping mechanism 60 and the stand 10 as a shaft, to rotate an angle corresponding to the input parameter, so that the included angle between the connecting line 72 of the axes of the first scraping shaft 61 and the second scraping shaft 62 and the vertical line "H" becomes "c" to realize the automatic adjustment function, and thus the first scraping shaft 61 and the second scraping shaft 62 generate a squeezing or scraping force corresponding to the input parameter on the film 90'. The method for processing other batches of films can be deduced by analogy.

## Claims

1. An apparatus (100) for processing a film (90), comprising a stand (10), a soaking tank (20), as well as a feeding shaft (30), a roll shaft (40), a stepping motor (50) and a scraping mechanism (60) mounted to the stand (10), respectively, wherein the roll shaft (40) can enter or go out of the soaking tank (20); the scraping mechanism (60) comprises a first scraping shaft (61) and a second scraping shaft (62) which are arranged side by side and spaced from each other at an appropriate distance so as to provide a space for passage of the film (90) between the first scraping shaft (61) and the second scraping shaft (62); the scraping mechanism (60) is connected to the stepping motor (50); an optocoupler switch (68) is fixed to the stand (10), and assists the stepping motor (50) to automatically find the initial position thereof; and then by taking the initial position as a basis, the stepping motor (50) drives, according to an input parameter, the scraping mechanism (60) to rotate an angle corresponding to the input parameter around the connecting shaft of the stepping motor (50) and the stand (10), thereby automatically adjusting the included angle between the connecting line of the axes of the first scraping shaft (61) and the second scraping shaft (62) and a vertical line;
wherein the appropriate distance provides a space for passage of the film (90) between the first scraping shaft (61) and the second scraping shaft (62), so that the upper and lower surfaces of the film (90) abut against the outer surfaces of the first scraping shaft (61) and the second scraping shaft (62), respectively, thus the first scraping shaft (61) and the second scraping shaft (62) generate a squeezing or scraping force corresponding to the input parameter on the film (90).

2. The apparatus (100) according to claim 1, wherein the optocoupler switch (68) comprises a U-shaped groove (69); the scraping mechanism (60) comprises an opaque paddle (70); and when the paddle (70) enters into the U-shaped groove (69), the optocoupler switch (68) is triggered to emit a signal, the stepping motor (50) stops running, and the first scraping shaft (61) and the second scraping shaft (62) are in initial positions.

3. The apparatus (100) according to claim 2, wherein the scraping mechanism (60) further comprises a synchronizing gear (65), the stepping motor (50) drives the scraping mechanism (60) to rotate together via the synchronizing gear (65), so as to adjust the included angle between the connecting line of the axes of the first scraping shaft (61) and the second scraping shaft (62) and the vertical line.

4. The apparatus (100) according to claim 3, wherein the paddle (70) is fixed to the side face of the synchronizing gear (65).

5. The apparatus (100) according to claim 4, wherein the feeding shaft (30) comprises two roll wheels (31, 32), at least one of which is connected with an elastic element (33) and swingable relative to the stand (10).

6. The apparatus (100) according to claim 4, wherein the feeding shaft (30) comprises two roll wheels (31, 32), and a counterweight element (39) movable up and down is disposed between the two roll wheels (31, 32).

7. The apparatus (100) according to claim 6, wherein the feeding shaft (30) further comprises a wing (38) extending downwards, a guide slot (37) is disposed on the wing (38), and the counterweight element (39) is located in the guide slot (37) and movable up and down in the guide slot (37).

8. The apparatus (100) according to claim 1, wherein the scraping mechanism (60) comprises a first baffle (63) and a second baffle (64), to which two ends of the first scraping shaft (61) and the second scraping shaft (62) are connected respectively; the first baffle (63) and the second baffle (64) are connected to the stand (10), respectively; the first baffle (63) is connected to the synchronizing gear (65) which is connected to the stepping motor (50); and under the driving of the stepping motor (50), the synchronizing gear (65) drives the first baffle (63) and the second baffle (64) together with the first scraping shaft (61) and the second scraping shaft (62) to rotate synchronously relative to the stand (10), so as to adjust the included angle between the connecting line of the axes of the first scraping shaft (61) and the second scraping shaft (62) and the vertical line.

9. The apparatus (100) according to one of claims 1 to 8, wherein the film (90) is used for manufacturing of test paper.

## Patentansprüche

1. Vorrichtung (100) zur Verarbeitung eines Films (90), umfassend einen Ständer (10), einen Einweichbehälter (20) sowie eine Zuführwelle (30), eine Walzenwelle (40), einen Schrittmotor (50) und einen Schabemechanismus (60) umfasst, die jeweils an dem Ständer (10) angebracht sind, wobei die Walzenwelle (40) in den Einweichbehälter (20) hinein- oder aus diesem herausgeführt werden kann; der Schabmechanismus (60) eine erste Schabwelle (61) und eine zweite Schabwelle (62) umfasst, die nebeneinander und in einem geeigneten Abstand voneinander angeordnet sind, um einen Durchgangsraum für den Film (90) zwischen der ersten Schabwelle (61) und der zweiten Schabwelle (62) zu schaffen; der Schabemechanismus (60) ist mit dem Schrittmotor (50) verbunden; ein Optokopplerschalter (68) ist an dem Ständer (10) befestigt und unterstützt den Schrittmotor (50) dabei, automatisch seine Ausgangsposition zu finden; und dann treibt der Schrittmotor (50) auf der Grundlage der Ausgangsposition gemäß einem Eingabeparameter den Schabemechanismus (60) an, sich um einen Winkel, der dem Eingabeparameter entspricht, um die Verbindungswelle des Schrittmotors (50) und des Ständers (10) zu drehen, wodurch automatisch der eingeschlossene Winkel zwischen der Verbindungslinie der Achsen der ersten Schabewelle (61) und der zweiten Schabewelle (62) und einer vertikalen Linie eingestellt wird;
wobei der geeignete Abstand einen Raum für den Durchgang des Fims (90) zwischen der ersten Schabwelle (61) und der zweiten Schabwelle (62) bereitstellt, sodass die Ober- und Unterseite des Films (90) jeweils an den Außenflächen der ersten Schabwelle (61) und der zweiten Schabwelle (62) anliegen, wodurch die erste Schabwelle (61) und die zweite Schabwelle (62) eine dem Eingabeparameter entsprechende Druck- oder Schabkraft auf den Film (90) ausüben.

2. Vorrichtung (100) nach Anspruch 1, wobei der Optokopplerschalter (68) eine U-förmige Nut (69) umfasst; der Schabemechanismus (60) ein undurchsichtiges Paddel (70) umfasst; und wenn das Paddel (70) in die U-förmige Nut (69) eintritt, der Optokopplerschalter (68) ausgelöst wird, um ein Signal auszusenden, der Schrittmotor (50) seinen Betrieb einstellt und die erste Schabewelle (61) und die zweite Schabewelle (62) sich in ihren Ausgangspositionen befinden.

3. Vorrichtung (100) nach Anspruch 2, wobei der Schabmechanismus (60) ferner ein Synchronisationsgetriebe (65) umfasst, wobei der Schrittmotor (50) den Schabemechanismus (60) über das Synchronisationsgetriebe (65) antreibt, um ihn mitzudrehen, um so den eingeschlossenen Winkel zwischen der Verbindungslinie der Achsen der ersten Schabewelle (61) und der zweiten Schabewelle (62) und der Vertikalen einzustellen.

4. Vorrichtung (100) nach Anspruch 3, wobei das Paddel (70) an der Seitenfläche des Synchronisationsgetriebes (65) befestigt ist.

5. Vorrichtung (100) nach Anspruch 4, wobei die Zuführwelle (30) zwei Laufräder (31, 32) umfasst, von denen mindestens eines mit einem elastischen Element (33) verbunden und relativ zum Ständer (10) schwenkbar ist.

6. Vorrichtung (100) nach Anspruch 4, wobei die Zuführwelle (30) zwei Laufräder (31, 32) umfasst und zwischen den beiden Laufrädern (31, 32) ein auf und ab bewegbares Gegengewichtselement (39) angeordnet ist.

7. Vorrichtung (100) nach Anspruch 6, wobei die Zuführwelle (30) ferner einen sich nach unten erstreckenden Flügel (38) umfasst, wobei ein Führungsschlitz (37) an dem Flügel (38) angeordnet ist und das Gegengewichtselement (39) in dem Führungsschlitz (37) angeordnet und in dem Führungsschlitz (37) auf und ab bewegbar ist.

8. Vorrichtung (100) nach Anspruch 1, wobei der Schabemechanismus (60) eine erste Prallplatte (63) und eine zweite Prallplatte (64) umfasst, mit denen jeweils zwei Enden der ersten Schabewelle (61) und der zweiten Schabewelle (62) verbunden sind; die erste Ablenkplatte (63) und die zweite Ablenkplatte (64) jeweils mit dem Ständer (10) verbunden sind; die erste Ablenkplatte (63) mit dem Synchronisationsgetriebe (65) verbunden ist, das mit dem Schrittmotor (50) verbunden ist; und unter dem Antrieb des Schrittmotors (50) treibt das Synchronisationsgetriebe (65) die erste Prallplatte (63) und die zweite Prallplatte (64) zusammen mit der ersten Schabwelle (61) und der zweiten Schabwelle (62) an, um sich synchron relativ zum Ständer (10) zu drehen, um den eingeschlossenen Winkel zwischen der Verbindungslinie der Achsen der ersten Schabwelle (61) und der zweiten Schabwelle (62) und der Vertikalen einzustellen.

9. Vorrichtung (100) nach einem der Ansprüche 1 bis 8, wobei der Film (90) zur Herstellung von Testpapier verwendet wird.

## Revendications

1. Appareil (100) pour traiter un film (90), comprenant un support (10), un réservoir de trempage (20), ainsi qu'un arbre d'alimentation (30), un arbre de rouleau (40), un moteur pas à pas (50) et un mécanisme de raclage (60) montés sur le support (10), respectivement, dans lequel l'arbre de rouleau (40) peut entrer ou sortir du réservoir de trempage (20) ; le mécanisme de raclage (60) comprend un premier arbre de raclage (61) et un second arbre de raclage (62) qui sont agencés côte à côte et espacés l'un de l'autre à une distance appropriée de manière à fournir un espace pour le passage du film (90) entre le premier arbre de raclage (61) et le second arbre de raclage (62) ; le mécanisme de raclage (60) est connecté au moteur pas à pas (50) ; un commutateur d'optocoupleur (68) est fixé au support (10), et aide le moteur pas à pas (50) à trouver automatiquement sa position initiale ; et ensuite en prenant la position initiale comme base, le moteur pas à pas (50) entraîne, selon un paramètre d'entrée, le mécanisme de raclage (60) pour faire tourner un angle correspondant au paramètre d'entrée autour de l'arbre de connexion du moteur pas à pas (50) et du support (10), ajustant ainsi automatiquement l'angle inclus entre la ligne de connexion des axes du premier arbre de raclage (61) et du second arbre de raclage (62) et une ligne verticale ;
dans lequel la distance appropriée fournit un espace pour le passage du film (90) entre le premier arbre de raclage (61) et le second arbre de raclage (62), de sorte que les surfaces supérieure et inférieure du film (90) viennent en butée contre les surfaces externes du premier arbre de raclage (61) et du second arbre de raclage (62), respectivement, de sorte que le premier arbre de raclage (61) et le second arbre de raclage (62) génèrent une force de serrage ou de raclage correspondant au paramètre d'entrée sur le film (90).

2. Appareil (100) selon la revendication 1, dans lequel le commutateur d'optocoupleur (68) comprend une rainure en forme de U (69) ; le mécanisme de raclage (60) comprend une palette opaque (70) ; et lorsque la palette (70) entre dans la rainure en forme de U (69), le commutateur d'optocoupleur (68) est déclenché pour émettre un signal, le moteur pas à pas (50) arrête de fonctionner, et le premier arbre de raclage (61) et le second arbre de raclage (62) sont dans des positions initiales.

3. Appareil (100) selon la revendication 2, dans lequel le mécanisme de raclage (60) comprend en outre un engrenage de synchronisation (65), le moteur pas à pas (50) entraîne le mécanisme de raclage (60) pour tourner ensemble via l'engrenage de synchronisation (65), de manière à ajuster l'angle inclus entre la ligne de connexion des axes du premier arbre de raclage (61) et du second arbre de raclage (62) et la ligne verticale.

4. Appareil (100) selon la revendication 3, dans lequel la palette (70) est fixée à la face latérale de l'engrenage de synchronisation (65).

5. Appareil (100) selon la revendication 4, dans lequel l'arbre d'alimentation (30) comprend deux roues de roulement (31, 32), dont au moins l'une est connectée à un élément élastique (33) et peut osciller par rapport au support (10).

6. Appareil (100) selon la revendication 4, dans lequel l'arbre d'alimentation (30) comprend deux roues de roulement (31, 32), et un élément de contrepoids (39) mobile vers le haut et vers le bas est disposé entre les deux roues de roulement (31, 32).

7. Appareil (100) selon la revendication 6, dans lequel l'arbre d'alimentation (30) comprend en outre une aile (38) s'étendant vers le bas, une fente de guidage (37) est disposée sur l'aile (38), et l'élément de contrepoids (39) est situé dans la fente de guidage (37) et mobile vers le haut et vers le bas dans la fente de guidage (37).

8. Appareil (100) selon la revendication 1, dans lequel le mécanisme de raclage (60) comprend un premier déflecteur (63) et un second déflecteur (64), auxquels deux extrémités du premier arbre de raclage (61) et du second arbre de raclage (62) sont connectées respectivement ; le premier déflecteur (63) et le second déflecteur (64) sont connectés au support (10), respectivement ; le premier déflecteur (63) est connecté à l'engrenage de synchronisation (65) qui est connecté au moteur pas à pas (50) ; et sous l'entraînement du moteur pas à pas (50), l'engrenage de synchronisation (65) entraîne le premier déflecteur (63) et le second déflecteur (64) ensemble avec le premier arbre de raclage (61) et le second arbre de raclage (62) pour tourner de manière synchrone par rapport au support (10), de manière à ajuster l'angle inclus entre la ligne de connexion des axes du premier arbre de raclage (61) et du second arbre de raclage (62) et la ligne verticale.

9. Appareil (100) selon l'une des revendications 1 à 8, dans lequel le film (90) est utilisé pour la fabrication de papier d'essai.
